# EUROPEAN PATENT APPLICATION

(11) **EP 3 943 111 A1**
(43) Date of publication of application: **26.01.2022**
(21) Application number: 21187460.7
(22) Date of filing: 23.07.2021
(51) Int. Cl.: A61K 41/00, C07K 16/44, G01N 33/577, G01N 33/68

(54) **ANTIBODIES FOR PHOTOACTIVE PROTEIN MANIPULATION**

(30) Priority: 24.07.2020 LT 2020539
(71) Applicant: Vilnius University, 01513 Vilnius (LT)
(72) Inventor: KLIMASAUSKAS, Saulius, Vilnius (LT); RAKAUSKAITE, Rasa, Vilnius (LT); URBANAVICIUTE, Giedr, Vilnius (LT); LASICKIENE, Rita, Vilnius (LT); SIMANAVICIUS, Martynas, Vilnius (LT); ZVIRBLIENE, Aurelija, Vilnius (LT)
(74) Representative: Petniunaite, Jurga

(57) **Abstract**

The invention describes a high affinity monoclonal antibody 12B6 that specifically interacts with a photolabile DMNB moiety. This antibody is suitable for recognizing DMNB groups in proteins (and potentially other biomolecules). The interaction of the antibody and the unique DMNB group allows the manipulation of proteins labeled with photolabile groups by immunoprecipitation.

## Description

### FIELD OF THE INVENTION

The invention relates to the immunological technologies. Its essence is the development of a high-affinity monoclonal antibody (MAb) that selectively interacts with the photosensitive 4,5-dimethoxy-2-nitrobenzyl (DMNB) group and its application for manipulating photoactive proteins.

### BACKGROUND ART

The engineering and synthesis of recombinant proteins with desirable catalytic properties in the cell is being widely developed for research into the structure and function of proteins and for practical application in medicine and bioindustry. Specifically, targeted modification of proteins provides a unique opportunity to precisely control their properties and expand their functions. Optical control of protein function creates a unique opportunity to control biological processes in time and space that cannot be created by other methods of conditional control. This is often achieved by the use of photolabile (FL) protecting groups, the removal of which by light is a gentle and non-invasive procedure. It is completely orthogonal to other chemical processes in the biological system. This technology has been successfully applied to control the photochemical activity of small molecules, peptides, oligonucleotides, and proteins (Spicer CD and Davis BG. Nat Commun. 2014; Sep 5;5:4740. doi: 10.1038/ncomms5740).

Several systems have been developed to allow the incorporation of photocaged lysine, tyrosine, serine, cysteine, and selenocysteine into the protein (Riggsbee CW and Deiters A. Trends Biotechnol. 2010;Sep;28(9):468-75; Dumas A et al. Chemical Science. 2015;6(1):50-69). The high nucleophilicity of the thiol in the cysteine residue has long been used in the implementation of various chemical functions. Cysteine (Cys) is a relatively uncommon amino acid, and free cysteines accessible to solvent in wild-type proteins are also relatively rare. Selenocysteine (Sec) - the 21 st amino acid - is even rarer and has even stronger functionality. Sec incorporated into proteins gives them special chemical properties (compared to Cys - high nucleophilicity, low pKa, low oxidation-reduction potential); Sec incorporated at the target protein position allows the naturally evolved catalytic capacity of the enzymes to be extended or creates sites for selective protein conjugation, labeling, dimerization, as well as altering protein folding. Sec is vital in organisms of all three domains that have developed the complex biosynthetic and translational mechanisms required to incorporate this rare amino acid into proteins. Despite these obvious advantages, few examples are described to illustrate an innovative and biologically compatible photochemical transformation. Thus, a new method that allows the selective introduction of photoactive chemical groups into proteins and their subsequent exploitation for practical purposes is a significant contribution to the biotechnology. Methods have been developed that allow the biosynthetic production of proteins with Cys protected by photosensitive group and introduced at genetically selected positions (Nguyen DP et al. J. Am. Chem. Soc. 2014;136:2240-3; Uprety R et al. ChemBioChem 2014;15:1793-9) or Sec (Rakauskaite R et al. Chem Commun (Camb) 2015;51(39):8245-8). The DMNB group protects highly reactive Sec (or solution-exposed Cys) from adverse side effects within protein-producing cells and during all protein manipulations and can be easily removed by irradiation with 370 nm light. Unfortunately, the purification of each new protein protected by FL group or its management in vitro or intracellularly requires an individual newly developed protocol, as there are currently no affinity ligands that specifically interact with a relatively small chemical group.

The present invention does not have the above-mentioned disadvantages associated with protein purification and includes additional advantages.

### SUMMARY OF INVENTION

To extend the overall applicability and functionality of this technology, we developed antibodies specific for the photo-protective DMNB group present in proteins. This new tool greatly facilitates intracellular studies of protected proteins (by immunoblotting) and affinity purification (by immunoprecipitation) specific to photoprotective group. We first developed an affinity-based purification system that purified a DMNB-labeled protein from complex molecular mixtures forming an immunoprecipitation complex. This method of protein purification can be further developed by using controlled irradiation of the immunoprecipitation complex, in which the protein is released without leaving traces of the photo tag. Similar systems would be highly desirable for the purification of recombinant selenoproteins when certain chemical reagents or conditions (imidazole, high concentration salt, thio-containing reducing agents, etc.) cannot be used. Such a strategy would also expand the diversity and availability of functionalized biomolecules through the deployment of miniaturized light-controlled systems, such as microfluidic devices, microarrays, and nanoparticles, whereas it would allow the selective accumulation of the labeled target protein on the surface of the particulate matter and the release of the target protein into solution by light at the desired time. The present invention, which combines molecular biology, bio-conjugation chemistry, photo-physics and immunology, would contribute to the technological progress of disciplines, such as protein sciences, synthetic biology and nanomedicine.

Although a number of methods have been developed to mark biomolecules with light-sensitive groups, there are currently no technologies in which the selective interaction of these groups with other molecules can be applied to the detection or enrichment of target proteins. To address this problem, we developed a high-affinity monoclonal antibody that selectively interacts with a photolabile chemical group and applied it to the manipulation of proteins containing such photolabile groups by light.

### SHORT DESCRIPTION OF DRAWINGS

Fig. 1 illustrates the identification of proteins with a photo-labile group by immunoblotting, using a monoclonal antibody.
Fig. 2 illustrates the detection limits of proteins with a photo-labile group by immunoblotting, using a monoclonal antibody.
Fig. 3 illustrates the detection of proteins with a photo-labile group by immunoblotting in complex molecular mixtures (cell lysates), using a monoclonal antibody.
Fig. 4 illustrates the immunoprecipitation (selective accumulation on the surface of particulate matter) of SUMOstar and EGFP proteins labeled with a photo-labile group at unique sites, using a monoclonal antibody.
Fig. 5 illustrates the immunoprecipitation (selective accumulation on the surface of particulate matter) of a unique SUMOstar protein labeled with a photo-labile group from a complex mixture of biomolecules, using a monoclonal antibody.
Fig. 6 illustrates the photochemical release of a protein labeled with a photo-labile group into the solution from an immunoprecipitation complex (surface of particulate matter).
Fig. 7 illustrates the identification of proteins photo-chemically released from the immunoprecipitation complex.

### DETAILED DESCRIPTION OF THE INVENTION

### Antigen preparation

Small chemical molecules - haptens - do not usually elicit a strong immune response. Conjugates of a non-immunogenic hapten and an immunogenic carrier protein are used to immunize animals when developing monoclonal antibodies against haptens (Chappey ON et al. Pharm Res. 1992; Nov;9(11):1375-9; Howard GC and Bethell DR. CRC Press, 2000).

To develop monoclonal antibodies specific for the DMNB compound, an immunization antigen, a conjugate of hapten (DMNB) and a carrier protein (KLH), was first prepared. DMNB bromide, which is reactive with sulfhydryls (-SH), was used for this purpose, and during the chemical reaction, DMNB groups were attached to free cysteine residues on the surface of the KLH protein. Cysteine is a relatively rare amino acid in proteins, so to increase the reactivity of the carrier protein with DMNB bromide, KLH was alternatively prepared with additional -SH residues. For this purpose, we used the primary amine sulfhydrylation reagent SATA (S-acetylthioacetate N-succinimide ester), which reacts with the lysine side chains of the protein. After reaction with hydroxylamine, the obtained deacylated KLH-AT carrier was conjugated analogously to DMNB.

The KLH-AT-DMNB antigen was further used to immunize mice, and the BSA-DMNB and BSA-AT-DMNB conjugates were used to assess antibody specificity.

Such a strategy was used to generate only DMNB group-specific antibodies that would not react with the carrier protein. The development of antibodies against chemical compounds (haptens) is a complex task because the chemical compounds themselves are non-immunogenic - they cannot activate the T-lymphocytes that are necessary for the activation of B-lymphocytes to produce antibodies with a high affinity IgG isotype. Since only protein antigens can activate T-lymphocytes, the hapten must be attached to a molecule of the carrier protein. It is essential to select the appropriate carrier protein and the appropriate method of hapten attachment so that the hapten is efficiently exposed to the surface and available to B-lymphocytes. This is particularly important for small haptens (<200 Da) because the molecular contact between the antibody and the hapten, which determines the selectivity and potency of the interaction, is minimal. The most efficient way of hapten exposure is difficult to predict and in each case is selected with the help of empirical experiments. Therefore, different ways of attaching the DMNB group were tested, through the SH group of the protein and through the SATA reagent. Different carrier proteins, KLH (for immunization) and BSA (for immune response screening and hybridoma screening), were used to select antibodies specific for the DMNB group only. Thus, high affinity antibodies were obtained, which did not show any cross-reactions with either the coupling reagent or the carrier protein.

### Development of a monoclonal antibody against a photo-labile DMNB group

A hybridoma cell line secreting a monoclonal antibody against DMNB was generated according to the methodology described previously (Howard GC and Bethell DR. CRC Press, 2000; Köhler G and Milstein C. Nature. 1975;Aug7;256(5517):495-7). Female BALB/c mice (6-8 weeks of age) were immunized with 50 µg of antigen with full Freud adjuvant for the first immunization, incomplete Freud adjuvant for the second immunization, and PBS for the third immunization. Subcutaneous injections were given 3 times every 28 days. The obtained hybridomas were cultured in selective HRT medium, DMNB-specific antibody-secreting hybrid cell clones were selected, and they were stabilized and cloned. After stabilization, the hybridoma clone 12B6 was selected, and the cells of this clone were frozen in cryopreservation medium and stored in liquid nitrogen.

The anti-DMNB monoclonal antibody (MAb) secreted by hybridoma 12B6 was characterized by using a commercial isotyping kit. This hybridoma was found to produce IgG class antibodies.

The hybridoma was deposited in Germany at the depository of the Leipzig Institute DSMZ on July 8, 2020, it was assigned the number DMNB12B6=DSM ACC3363.

### Specificity analysis of monoclonal antibody 12B6

Anti-DMNB MAb 12B6 was characterized by using classical enzyme-linked immunosorbent assay and immunoblotting techniques (Howard GC and Bethell DR. CRC Press, 2000). The specificity of the anti-DMNB antibody for the DMNB group was determined by enzyme-linked immunosorbent assay by using the immunization antigen KLH-AT-DMNB and the non-immunization conjugates BSA-DMNB and BSA-AT-DMNB.

After assessing the affinity of DMNB-specific MAb by indirect enzyme-linked immunosorbent assay, the apparent dissociation constant parameter value of MAb 12B6 was found to be 1.40×10⁻¹⁰ M.

Antibody 12B6 not only interacted well with multiple groups of labeled KLH and BSA proteins, but also with EGFP Y39DMNB-C containing a single DMNB group, resulting in clear immunoblotting signals. The antibody did not interact at all with the same unlabeled proteins, or proteins in which the DMNB group was removed by light.

Antibody 12B6 was purified by affinity chromatography by using Sepharose with attached protein A.

### EMBODIMENTS OF THE INVENTION

### Example 1. Detection of proteins protected by photo-labile group by immunoblotting

The anti-DMNB 12B6 antibody allows the specific determination of DMNB groups in proteins when the test proteins are denatured with SDS solution under PAA gel electrophoresis conditions. Various commercial (KLH, BSA, lysozyme), model (EGFP, SUMOstar) and unique laboratory synthesized proteins (DNA methyltransferases: M.Hpall, M.HhaIΔHT, M2.Eco31I) were used for these studies. Two kinds of proteins were prepared for analysis: 1) chemically labeled with multiple DMNB groups and 2) biosynthetic proteins with a single DMNB group. Multiple groups of DMNB were introduced into 2 commercial and 14 laboratory-synthesized and Ni-IMAC-purified proteins by chemical reaction with DMNB bromide. A single DMNB group was introduced into 13 proteins biosynthetically by using genetically encoded non-standard amino acid insertion technology (Rakauskaite R et al. Chem Commun (Camb). 2015;51(39):8245-8). They were purified by affinity Ni-IMAC chromatography. Molecular mass and the number of attached DMNB groups were confirmed by HPLC-ESI/MS analysis for all labeled proteins.

The immunoblotting method was used to detect proteins labeled with both multiple and single DMNB groups. For all proteins tested, the 12B6 antibody interacted with DMNB and allowed specific identification of labeled proteins.

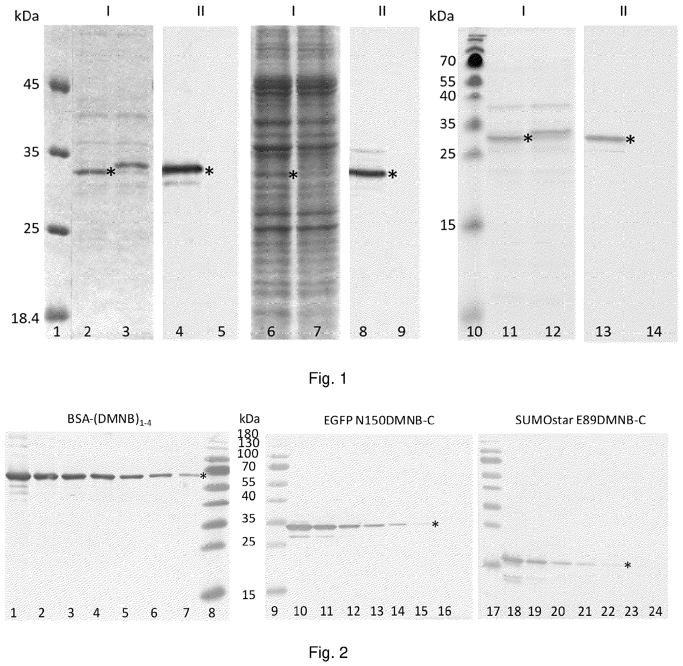

Fig. 1 shows that a unique photolabile group attached to both S and Se atoms (EGFP Y39DMNB-C and EGFP Y39DMNB-U proteins, respectively) is recognized by immunoblotting with high specificity when using anti-DMNB antibody 12B6: upon exposure to DMNB-protein at 365 nm light, the DMNB group is removed and the immunoblotting signal is lost. Synthesis of EGFP Y39DMNB-C and EGFP Y39DMNB-U proteins, preparation of cell lysate soluble fraction, protein purification, characterization and photochemical cleavage reaction of DMNB group were performed according to the previously described methodology (Rakauskaite R et al. Chem Commun (Camb). 2015;51(39):8245-8). Protein preparations of purified EGFP Y39DMNB-C (lanes 2-5) and EGFP Y39DMNB-U (lanes 11-14) were used in the experiment, as well as a complex molecular mixture of soluble fraction of yeast cells synthesizing EGFP Y39DMNB-C (lanes 6- 9). Protein samples were fractionated in two identical SDS-PAA gels, one (I) stained with Coomassie blue and the other (II) analyzed by immunoblotting by using anti-DMNB 12B6 antibody. Recombinant DMNB-proteins are marked with an asterisk.
Lanes 1, 10. Protein molecular weight standard.
Lane 2. Purified EGFP Y39DMNB-C (SDS-PAA gel stained with Coomassie blue).
Lane 3. Purified EGFP Y39DMNB-C, treated with UV (SDS-PAA gel stained with Coomassie blue).
Lane 4. Purified EGFP Y39DMNB-C (immunoblotting analysis using 12B6 MAb).
Lane 5. Purified EGFP Y39DMNB-C, treated with UV (immunoblotting analysis using 12B6 MAb).
Lane 6. Soluble fraction of extract of cells synthesizing EGFP Y39DMNB-C (SDS-PAA gel stained with Coomassie blue).
Lane 7. Soluble fraction of extract of cells synthesizing EGFP Y39DMNB-C, treated with UV (SDS-PAA gel stained with Coomassie blue).
Lane 8. Soluble fraction of extract of cells synthesizing EGFP Y39DMNB-C (immunoblotting analysis using 12B6 MAb).
Lane 9. Soluble fraction of extract of cells synthesizing EGFP Y39DMNB-C, treated with UV (immunoblotting analysis using 12B6 MAb).
Lane 11. Purified EGFP Y39DMNB-U (SDS-PAA gel stained with Coomassie blue).
Lane 12. Purified EGFP Y39DMNB-U, treated with UV (SDS-PAA gel stained with Coomassie blue).
Lane 13. Purified EGFP Y39DMNB-U (immunoblotting analysis using 12B6 MAb).
Lane 14. Purified EGFP Y39DMNB-U, treated with UV (immunoblotting analysis using 12B6 MAb).

### Example 2. Detection efficiency of proteins protected by photo-labile group by immunoblotting

The limit of detection of DMNB-proteins was assessed by immunoblotting. For this purpose, we performed detection of purified model DMNB-proteins by using serial protein dilutions (Fig. 2). The following experiments determined the lowest detectable amounts of protein: 16 ng BSA-(DMNB)₁₋₄, 31 ng EGFP-(DMNB)i and 63 ng SUMOstar-(DMNB)₁.
Lane 1. BSA-(DMNB)₁₋₄, 1 µg.
Lane 2. BSA-(DMNB)₁₋₄, 0.5 µg.
Lane 3. BSA-(DMNB)₁₋₄, 0.25 µg.
Lane 4. BSA-(DMNB)₁₋₄, 0.125 µg.
Lane 5. BSA-(DMNB)₁₋₄, 0.063 µg.
Lane 6. BSA-(DMNB)₁₋₄, 0.031 µg.
Lane 7. BSA-(DMNB)₁₋₄, 0.016 µg.
Lanes 8, 9, 17. Protein molecular weight standard.
Lane 10. EGFP N150DMNB-C, 1 µg.
Lane 11. EGFP N150DMNB-C, 0.5 µg.
Lane 12. EGFP N150DMNB-C, 0.25 µg.
Lane 13. EGFP N150DMNB-C, 0.125 µg.
Lane 14. EGFP N150DMNB-C, 0.063 µg.
Lane 15. EGFP N150DMNB-C, 0.031 µg.
Lane 16. EGFP N150DMNB-C, 0.016 µg.
Lane 18. SUMOstar E89DMNB-C, 1 µg.
Lane 19. SUMOstar E89DMNB-C, 0.5 µg.
Lane 20. SUMOstar E89DMNB-C, 0.25 µg.
Lane 21. SUMOstar E89DMNB-C, 0.125 µg.
Lane 22. SUMOstar E89DMNB-C, 0.063 µg.
Lane 23. SUMOstar E89DMNB-C, 0.031 µg.
Lane 24. SUMOstar E89DMNB-C, 0.016 µg.

### Example 3. Detection of proteins protected by photo-labile group in cell lysates by immunoblotting

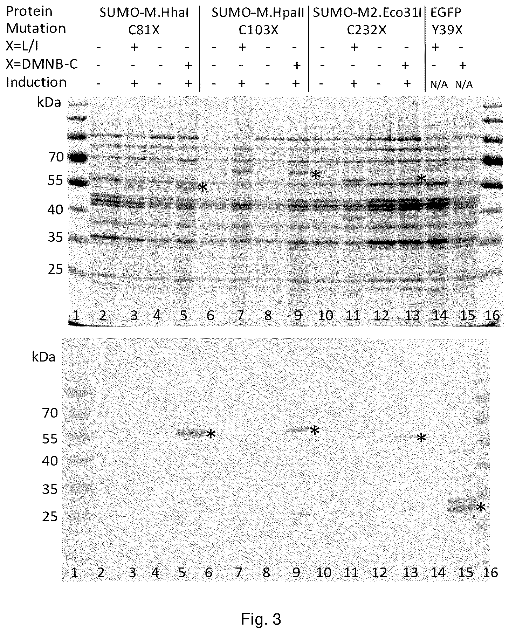

The anti-DMNB monoclonal antibody12B6 allows the identification of various proteins labeled with a single DMNB group in complex molecular mixtures (cell lysates) (Fig. 3). The biosynthesis of the four model proteins used in these experiments took place in yeast S. *cerevisiae* cells: expression of SUMO fusion proteins M.Hhal, M.Hpall, and M2.Eco31I was induced by CuSO₄; EGFP protein was expressed from the constitutional ADH1 promoter (N/A-induction conditions do not apply). The photochemical DMNB group was introduced into recombinant proteins at indicated positions by the method of genetically encoded non-standard amino acids (Rakauskaite R et al. Chem Commun (Camb), 2015;51(39): 8245-8) supplying cell growth medium with DMNB-C (lanes 5, 9, 13, 15). A leucine (L) or isoleucine (I) mutation (lanes 3, 7, 11, 14) was introduced at the same positions in the corresponding reference recombinant proteins. Biomasses of cells synthesizing recombinant proteins were harvested and lysates prepared from them were fractionated on an SDS-PAA gel (top panel: The SDS-PAA gel was stained with Coomassie blue, the recombinant proteins are marked with an asterisk). The DMNB proteins in the lysates were identified in an analogous SDS-PAA gel by immunoblotting using antibody 12B6 (bottom panel). Only DMNB-containing proteins were specifically identified by immunoblotting. The other components of the cell lysate did not react with MAb 12B6.
Lanes 1, 16 - protein size standard.
Lane 2. SUMOstar-M.Hhal C81L/I protein expression not induced.
Lane 3. SUMOstar-M.Hhal C81L/I protein expression induced.
Lane 4. SUMOstar-M.Hhal C81DMNB-C protein expression not induced.
Lane 5. SUMOstar-M.Hhal C81DMNB-C protein expression induced.
Lane 6. SUMOstar-M.Hpall C103L/I protein expression not induced.
Lane 7. SUMOstar-M.Hpall C103L/I protein expression induced.
Lane 8. SUMOstar-M.Hpall C103DMNB-C protein expression not induced.
Lane 9. SUMOstar-M.Hpall C103DMNB-C protein expression induced.
Lane 10. SUMOstar-M2.Eco31I C232L/I protein expression not induced.
Lane 11. SUMOstar-M2.Eco31I C232L/I protein expression induced.
Lane 12. SUMOstar-M2.Eco31I C232DMNB-C protein expression not induced.
Lane 13. SUMOstar-M2.Eco31I C232DMNB-C protein expression induced.
Lane 14. EGFP Y39L/I protein expression.
Lane 15. EGFP Y39DMNB-C protein expression.

### Example 4. Isolation of proteins labeled with the photo-labile group by immunoprecipitation

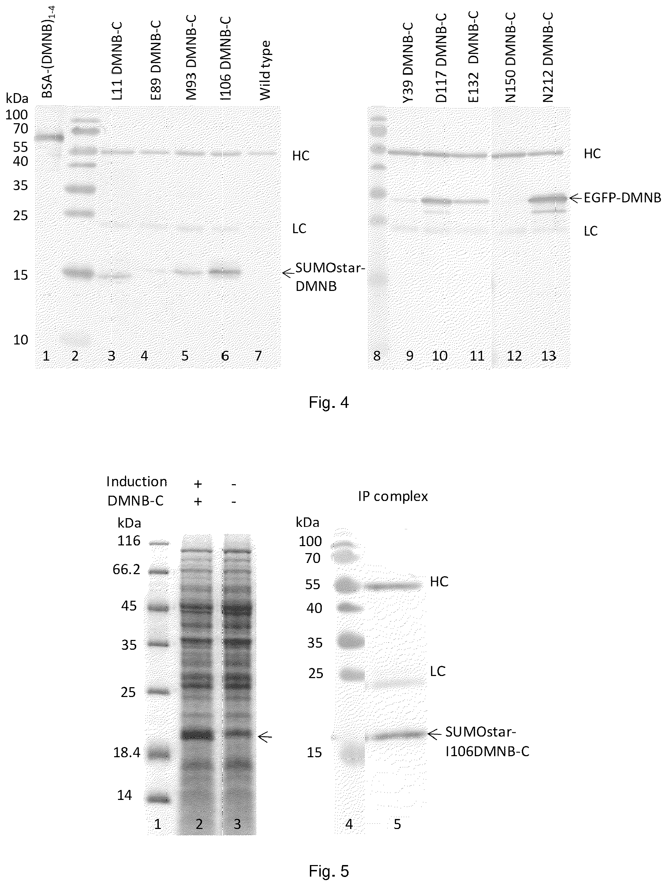

An immunoprecipitation experiment using DMNB-labeled proteins indicates that the anti-DMNB monoclonal antibody 12B6 is suitable for the recognition of native proteins. Although the photochemical group of DMNB is a small hapten, its interaction with MAb provides a stable immunoprecipitation (IP) complex (Fig. 4).

Magnetic beads (MagnaBind protein A beads, Thermofisher) with attached protein A, which specifically interacts with IgG class immunoglobulins, were used to form IP complexes. Magnetic beads with attached protein A were first incubated with MAb 12B6, and further used for incubation with DMNB-proteins. IP complexes were formed by using purified, dissolved in PBS buffer SUMOstar and EGFP proteins carrying non-canonical amino acid S-DMNB-cysteine at unique surface positions (SUMOstar: L11, E89, M93, 1106; EGFP: Y39, D117, E132, N150, N212). After incubation with proteins, the magnetic beads were washed with PBS. The proteins from the beads were released into solution by incubating the beads in denaturing SDS-PAGE sample buffer for 10 min at 95°C, fractionated on an SDS-PAA gel, and analyzed by immunoblotting using anti-DMNB antibody 12B6. It should be noted that in addition to DMNB-proteins, the 12B6 antibody chains released from the IP complexes, heavy (HC) and light (LC), are visualized due to the interaction with secondary antibodies. Three of the four SUMOstar-DMNB analyzed and three of the five EGFP-DMNB protein variants showed good or very good IP efficiency (lanes 3, 5, 6, 10, 11, and 13). These experiments suggest that IP may occur in the presence of a DMNB label at various protein sites.
Lane 1. Chemically DMNB-labeled BSA-(DMNB)₁₋₄ (immunoblotting control).
Lanes 2, 8 ― protein-size standard.
Lane 3. SUMOstar L11DMNB-C IP complex.
Lane 4. SUMOstar E89DMNB-C IP complex.
Lane 5. SUMOstar M93DMNB-C IP complex.
Lane 6. SUMOstar I106DMNB-C IP complex.
Lane 7. SUMOstar (wild type) IP complex.
Lane 9. EGFP Y39DMNB-C IP complex.
Lane 10. EGFP D117DMNB-C IP complex.
Lane 11. EGFP E132DMNB-C IP complex.
Lane 12. EGFP N150DMNB-C IP complex.
Lane 13. EGFP N212DMNB-C IP complex.

### Example 5. Purification of proteins by immunoprecipitation from complex molecular mixtures

During immunoprecipitation, MAb 12B6 specifically interacts only with DMNB groups present in proteins and does not form non-specific interactions with other cell lysate proteins. The SUMOstar I106DMNB-C protein, which forms the IP complex in a best way (Fig. 4), was also efficiently purified from yeast cell lysate (Fig. 5).

The photochemical DMNB group was introduced into the recombinant protein SUMOstar I106DMNB-C by the method of genetically encoded non-standard amino acids (Rakauskaite R et al. Chem Commun (Camb) 2015;51(39):8245-8), supplying cell growth medium with DMNB-C. Recombinant protein expression was confirmed by SDS-PAGE analysis by fractionating lysates of CuSO₄-induced (lane 2) and non-induced (lane 3) cells. A lysate of induced yeast cells was prepared for the IP experiment and further incubated with magnetic beads to which protein A and MAb 12B6 were attached. Magnetic beads were collected and target protein SUMOstar I106DMNB-C as well as MAb 12B6 were visualized by immunoblotting (lane 5) as described in Example 4.
Lanes 1, 4 - protein size standard.
Lane 2. SUMOstar I106DMNB-C expression induced (SDS-PAA gel stained with Coomassie blue).
Lane 3. SUMOstar I106DMNB-C expression not induced (SDS-PAA gel stained with Coomassie blue).
Lane 5. SUMOstar I106DMNB-C IP complex (immunoblotting analysis using MAb 12B6).

### Example 6. Photochemical release of a protein from an immunoprecipitation complex

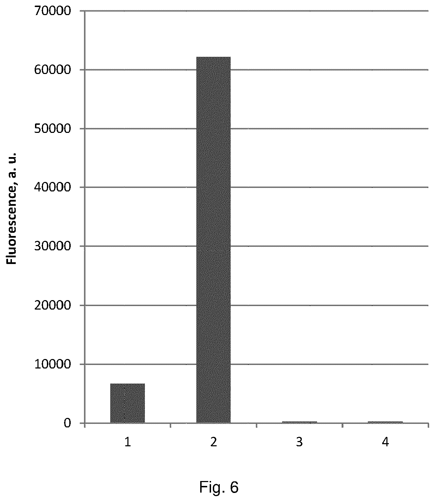

During the photochemical reaction, when the IP complexes are exposed to UV light, the DMNB protecting group is cleaved from the DMNB-proteins in the complexes and proteins without traces of the chemical label are released into the solution. EGFP proteins were chosen for the experiment because their photochemical release from the IP complex can be indirectly observed as an increase in the fluorescence of the solution after UV exposure (Fig. 6). For the photochemical reaction, an IP complex was prepared consisting of silica particles (Silica Particles, Protein A coated, 1 µm; Kisker) to which protein A, 12B6 antibody, and chemically labeled EGFP D117DMNB-C antigen (columns 1, 2) was attached. Similar control IP complex was prepared by using unlabeled EGFP D117C protein (columns 3, 4). Two samples of each complex were taken for the experiment (65 µl of a 0.3% suspension of IP particles in PBS buffer): one sample (columns 1, 3) was kept in the dark at 4°C and the other samples (columns 2, 4) were irradiated by 365 nm, for 10 min, at 4°C. Particles from all samples were separated and the remaining solutions were used to assess EGFP fluorescence. Exposure of EGFP D117DMNB-C IP complex to light increased the fluorescence of the solution by 9.3-fold compared to the non-irradiated complex (columns 1 and 2), whereas for control EGFP D117C IP complex only slight background fluorescence was observed in both conditions (columns 3 and 4).
Column 1. EGFP D117DMNB-C IP complex, unexposed to UV.
Column 2. EGFP D117DMNB-C IP complex, exposed to UV.
Column 3. EGFP D117C IP complex, unexposed to UV.
Column 4. EGFP D117C IP complex, exposed to UV.

### Example 7. Identification of proteins photochemically released from the immunoprecipitation complex

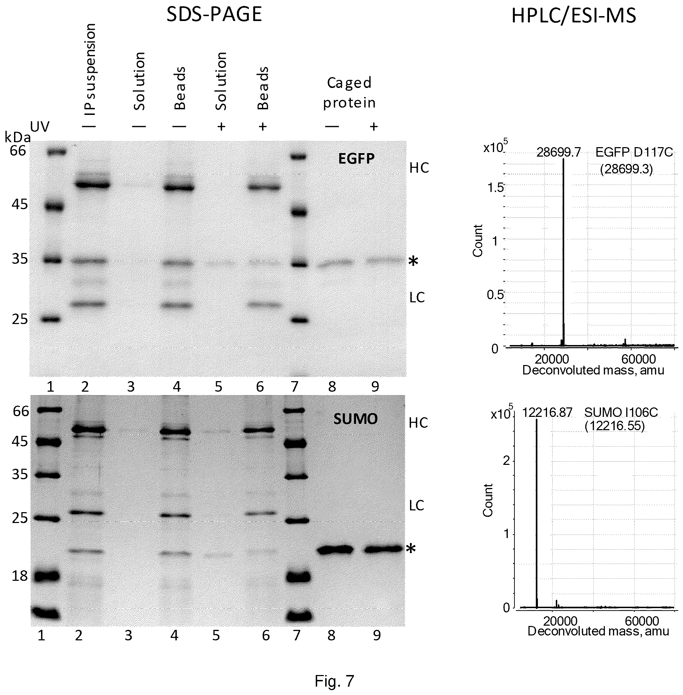

After exposure of IP complexes to UV light, EGFP D117C and SUMOstar I106C proteins released into the solution and having lost the DNMB label were identified by SDS-PAA gel fractionation and mass spectrometry (HPLC/ESI-MS) methods (Figure 7). Immunoprecipitation complexes were formed by using silica particles conjugated to protein A (Silica Particles, Protein A coated, 1 µm; Kisker) and incubated with 12B6 antibody. These particles were further incubated with a solution of purified EGFP D117C-(DMNB)₁₋₃ protein (Fig. 7, Lane 2 top panel) or cell lysate of yeast expressing SUMOstar I106C-DMNB protein (Fig. 7, Lane 2 bottom panel). A solution and particles of reference samples (lanes 3, 4) and 365 nm light-exposed samples (lanes 5, 6) were separated, fractionated on SDS-PAA gel, and stained with Coomassie blue. During the analysis, the signal of the analyzed proteins in the "solution" sample was observed due to UV irradiation (lane 5), while in the "particle" sample (lane 6) this signal was attenuated. Control photochemical reactions were performed by using sole solutions of pure EGFP D117C-(DMNB)₁₋₃ (Fig. 7, Lanes 8, 9 top panel) and SUMOstar I106C-(DMNB)₁₋₂ (Fig. 7, Lanes 8, 9 bottom panel) proteins and shows that UV exposure did not have a significant effect on the proteins analyzed. The bands of the target proteins are marked with an asterisk. HC, heavy chain. LC, light chain. M, protein size standard.

The EGFP D117C and SUMOstar I106C proteins that lost DMNB label during the photochemical reaction and were released into the solution (Fig. 7, Lane 5) were directly identified by mass spectrometry (HPLC/ESI-MS) (Fig. 7). Calculated protein masses are indicated in parentheses.
Lanes 1, 7 - protein size standard.
Lane 2. IP suspension.
Lane 3. IP suspension solution unexposed to UV.
Lane 4. IP suspension particles unexposed to UV.
Lane 5. IP suspension solution exposed to UV.
Lane 6. IP suspension particles exposed to UV.
Lane 8. DMNB-protein unexposed to UV.
Lane 9. DMNB-protein exposed to UV.

## Claims

1. A monoclonal antibody **characterized in that** it specifically interacts with a photolabile chemical group of DMNB.

2. The monoclonal antibody according to claim 1, **characterized in that** it has a high affinity.

3. The monoclonal antibody according to claims 1-2, **characterized in that** the apparent value of the dissociation constant is 1.40 × 10⁻¹⁰ M.

4. The monoclonal antibody according to claims 1-3, **characterized in that** it specifically binds to the DMNB group in the protein.

5. The monoclonal antibody according to claims 1-4, **characterized in that** the lowest limit of detection for DMNB-proteins is >16 ng.

6. The monoclonal antibody according to claims 1-5, **characterized in that** it identifies various proteins labeled with a single DMNB moiety in complex molecular mixtures, such as cell lysates.

7. The monoclonal antibody according to claims 1-6, **characterized in that** it provides a stable immunoprecipitation complex with a DMNB tag at various protein sites.

8. The monoclonal antibody according to claims 1-7, **characterized in that** it is produced by 12B6 hybridoma deposited at DSMZ under the number: DSM ACC3363.

9. Use of a monoclonal antibody according to claims 1-8 for the identification of labeled proteins.

10. Use of a monoclonal antibody according to claims 1-8 for the accumulation of proteins having photolabile groups on the surface of particulate matter and for their release into solution by means of light.

11. Use of a monoclonal antibody according to claims 1-8 in a method of protein purification, **characterized in that** the monoclonal antibody specifically interacts only with DMNB groups present in the protein and does not form non-specific interactions with other cell lysate proteins.

12. The method of protein purification according to claim 11, **characterized in that** the DMNB-labeled protein is purified from complex molecular mixtures.

13. The method of protein purification according to claim 11, **characterized in that** during the photochemical reaction, the DMNB protecting group is cleaved from the proteins by exposing the immunoprecipitation complexes to UV light and the proteins are released into the solution without traces of chemical label.
